# EUROPÄISCHE PATENTSCHRIFT

(11) **EP 1 174 128 B1**
(45) Veröffentlichungstag und Bekanntmachung des Hinweises auf die Patenterteilung: **08.01.2003**
(21) Anmeldenummer: 01116605.5
(22) Anmeldetag: 11.07.2001
(51) Int. Cl.: A61K 9/50, A61K 31/375, A61P 37/04

(54) **Vitamin-C-Präparat und Verfahren zu dessen Herstellung**
Vitamin-C preparation and process for preparation thereof
Préparation de vitamine C et son procédé

(30) Priorität: 17.07.2000 DE 10035088
(43) Veröffentlichungstag der Anmeldung: 23.01.2002
(73) Patentinhaber: Von Rhein, Wolfgang, 45134 Essen (DE)
(72) Erfinder: Von Rhein, Wolfgang, 45134 Essen (DE)
(74) Vertreter: Zenz, Joachim Klaus, Dipl.-Ing.

(56) Entgegenhaltungen:
- US-A- 4 605 666
- US-A- 5 885 547
- KAWASHIMA Y: "HOLLOW MICROSPHERES FOR USE AS A FLOATING CONTROLLED DRUG DELIVERY SYSTEM IN THE STOMACH" JOURNAL OF PHARMACEUTICAL SCIENCES, AMERICAN PHARMACEUTICAL ASSOCIATION. WASHINGTON, US, Bd. 81, Nr. 2, 1. Februar 1992 (1992-02-01), Seiten 135-140, XP000248776 ISSN: 0022-3549

## Beschreibung

Vitamin-C-Präparate sind derzeit in Form von kristalliner Ascorbinsäure oder von gepreßten Tabletten im Handel erhältlich. Die Einnahme derartiger Vitamin-C-Gaben erfolgt entweder als Mischung kristalliner Ascorbinsäure mit Zucker oder nach Auflösung einer verpreßten Tablette in Wasser als sprudelndes Vitamin-C-Getränk.

Aus der WO 98/33397 ist ein Vitamin-C-Präparat bekannt, bei dem Ascorbinsäurekristalle in Wasser gelöst und in einen pastösen Zustand geliert werden. Das Vitamin-C-haltige Gel wird in eine Tube aus plastisch verformbarem Staniol verfüllt. In dieser Tube hat das gelförmige Vitamin-C-Präparat einen dauerhaften Schutz vor Licht und Oxidation und ist dementsprechend lange haltbar, selbst wenn die Tube teilweise oder weitgehend entleert worden ist. Dieses gelförmige Vitamin-C-Präparat kann in einfacher Form Speisen zugegeben werden, um den Vitamin-C-Bedarf zu decken. Bei vielen Speisen und Speisezutaten trägt das bekannte gelförmige Vitaminpräparat zu einer säuerlichen Geschmacksnote bei, die den Eindruck der Frische erzeugt und damit der Speise eine bessere Geschmacksnote verleiht.

Die Erfindung geht von der Überlegung aus, daß Vitamin-C als partikelförmiges Präparat dem Konsumenten vertrauter und angenehmer ist, da er die Handhabung und Dosierung von partikelförmigen Würzen kennt.

Der Erfindung liegt die Aufgabe zugrunde, ein Vitamin-C-Präparat in gebrauchsfertiger Form zur Verfügung zu stellen, das mühelos dosiert und Speisen zugeführt werden kann und in wasserhaltigen, für Speisen also typischen Lösungsmitteln äußerst rasch in Lösung geht.

Das erfindungsgemäße Vitamin-C-Präparat, das aus Mikropartikeln aus im wesentlichen reiner Ascorbinsäure besteht, ist dadurch gekennzeichnet, daß die Mikropartikel als Bläschen oder Hohlkügelchen ausgebildet sind, deren Durchmesser kleiner als 0,2 mm ist, wobei die Mikropartikel ein Verhältnis von Feststoffmasse zu -oberfläche kleiner als 50% dieses Verhältnisses bei kristalliner Ascorbinsäure haben.

Diese hohlkugelartige Ausbildung der Mikropartikel führt zu einer sprunghaften Erhöhung der relativen Oberfläche, also zu einer Verringerung des Masse/Oberflächenverhältnisses gegenüber kristallinen Ascorbinsäurepartikeln. Dies wiederum hat zur Folge, daß sich die Ascorbinsäure extrem rasch und mit optimaler Verteilung auflöst und die als Träger dienende Speise eine einheitliche und frische Geschmacksnote erhält. Das neue Vitamin-C-Präparat eröffnet daher bisher unerreichte Nutzungsmöglichkeiten insbesondere als tischfertiger Speisezusatz, der sowohl Süßspeisen als auch salziger oder neutral schmeckender Kost eine zumeist beliebte säuerliche Geschmacksnote verleiht.

Die Ascorbinsäure-Bläschen oder -hohlkügelchen sind in der Regel gasgefüllt, vorzugsweise luftgefüllt. Ihr Durchmesser liegt in der Regel zwischen 5 und 100 µm, vorzugsweise zwischen 8 und 50 µm.

Gemäß einem bevorzugten Ausführungsbeispiel sind mehrere Ascorbinsäurebläschen oder -hohlkügelchen unter weitgehender Beibehaltung ihrer Bläschen- oder -Hohlkugelform zu größeren Aggregaten granuliert, wobei die Granulate vorzugsweise einen Maximaldurchmesser kleiner 0,5 mm haben. Kleinstückige Granulate lassen sich nämlich über eine Streuvorrichtung auf der Speise gut verteilen und sind ähnlich kristallinen Salzkörnern oder gemahlenem Pfeffer in bekannter Weise handhabbar. Die Weiterverarbeitung zu Granulaten ändert an dem günstigen Verhältnis von Feststoffmasse zu Oberfläche (große relative Oberfläche) nicht oder wenig, wirkt aber einem Verklumpen der Bläschen oder Hohlkügelchen entgegen und verbessert damit die Streufähigkeit und Verteilbarkeit des Vitamin-C-Präparats.

Das erfindungsgemäße Verfahren zur Herstellung des neuen Vitamin-C-Präparates zeichnet sich dadurch aus, daß kristalline Ascorbinsäure in einem flüssigen Lösungsmittel gelöst und danach einer Sprühtrocknung unterworfen wird, wobei die Lösung feinst versprüht und das flüssige Lösungsmittel verdunstet wird, daß danach entstehende feinste Bläschen oder Hohlkügelchen aufgefangen und in Behältern im wesentlichen luftdicht und lichtgeschützt abgefüllt werden.

Die Lösung der kristallinen Ascorbinsäure erfolgt in einem wässrigen Lösungsmittel. Als wässriges Lösungsmittel dient gemäß einem bevorzugten Beispiel der Erfindung destilliertes Wasser.

Die Sprühtrocknung erfolgt vorzugsweise im Gleichstromverfahren, wobei die der Trocknung dienende Luft und die feinst versprühte ascorbinsäurehaltige Lösung in einer geeigneten Mischkammer in im wesentlichen gleichen Richtungen geführt werden.

Eine alternative Möglichkeit der Sprühtrocknung besteht darin, die ascorbinsäurehaltige wässrige Lösung in einer auf niedrigem Druck befindliche Kammer einzudüsen und zu verdampfen.

Die bei der Sprühtrocknung anfallenden feinen Ascorbinsäurebläschen bzw. -hohlkügelchen werden vor dem Abfüllen vorzugsweise zu größeren Aggregaten vereinigt, wobei sie an ihren Außenhäuten miteinander verschmolzen werden. Dies kann beispielsweise dadurch geschehen, daß während, am Ende oder nach der Sprühtrocknung ein geeignetes Bindemittel zugegeben wird, das die Außenhäute der Ascorbinsäurebläschen oder - hohlkügelchen anlöst. Als Bindemittel eignet sich beispielsweise eine Stärkelösung. Die Größe der Agglomerate kann durch Dosierung des Bindemittels eingestellt werden. Unerwünscht große Agglomerate können abgesiebt werden, bevor die agglomerierten Bläschen oder Hohlkügelchen in einem Behälter, beispielsweise einer lichtgeschützten und im wesentlichen luftdichten Flasche abgefüllt werden. Im Flaschenhals kann an einer geeigneten Schraubkappe ein Sieb eingebaut sein, mit dessen Hilfe das Vitamin-C-Agglomerat nach Art eines herkömmlichen Salzspenders auf der Speise verteilt werden kann.

## Patentansprüche

1. Vitamin-C-Präparat, bestehend aus Mikropartikeln aus im wesentlichen reiner Ascorbinsäure,
**dadurch gekennzeichnet,**
**daß** die Mikropartikel als Bläschen oder Hohlkügelchen ausgebildet sind, deren Durchmesser kleiner als 0,2 mm ist, und ein Verhältnis von Feststoffmasse zu -oberfläche kleiner als 50% dieses Verhältnisses bei kristalliner Ascorbinsäure haben.

2. Vitamin-C-Präparat nach Anspruch 1, **dadurch gekennzeichnet, daß** die Ascorbinsäure-Bläschen oder -Hohlkügelchen gasgefüllt sind.

3. Vitamin-C-Präparat nach Anspruch 1 oder 2, **dadurch gekennzeichnet, daß** die Bläschen oder Hohlkügelchen einen Durchmesser kleiner als 0,1 mm haben.

4. Vitamin-C-Präparat nach einem der Ansprüche 1 bis 3, **dadurch gekennzeichnet, daß** mehrere Bläschen oder Hohlkügelchen unter weitgehender Beibehaltung ihrer Bläschen- oder Hohlkugelform zu größeren Aggregaten granuliert sind.

5. Vitamin-C-Präparate nach Anspruch 4, **dadurch gekennzeichnet, daß** die Granulate einen Maximaldurchmesser kleiner 0,5 mm haben.

6. Verfahren zur Herstellung eines Vitamin-C-Präparates nach einem der Ansprüche 1 bis 5, **dadurch gekennzeichnet, daß** kristalline Ascorbinsäure in einem flüssigen Lösungsmittel gelöst und danach einer Sprühtrocknung unterworfen wird, wobei die Lösung feinst versprüht, und das flüssige Lösungsmittel verdunstet wird, daß danach entstehende feine Bläschen oder Hohlkügelchen aufgefangen und in Behältern im wesentlichen luftdicht und lichtgeschützt abgefüllt werden.

7. Verfahren nach Anspruch 6, **dadurch gekennzeichnet, daß** die bei der Sprühtrocknung anfallenden feinen Bläschen bzw. Hohlkügelchen vor dem Abfüllen zu größeren Aggregaten vereinigt werden, wobei sie an ihren Außenhäuten miteinander verschmolzen werden.

8. Verfahren nach Anspruch 7, **dadurch gekennzeichnet, daß** die feinen Ascorbinsäurebläschen oder -hohlkügelchen durch Zugabe eines Bindemittels agglomeriert werden.

9. Verfahren nach Anspruch 8, **dadurch gekennzeichnet, daß** die Ascorbinsäurebläschen oder -hohlkügelchen zum Agglomerieren mit einer Stärkelösung besprüht werden.

10. Verfahren nach Anspruch 6 bis 9, **dadurch gekennzeichnet, daß** die kristalline Ascorbinsäure in einem wässrigen Lösungsmittel gelöst wird.

11. Verfahren nach Anspruch 10, **dadurch gekennzeichnet, daß** als wässriges Lösungsmittel destilliertes Wasser verwendet wird.

12. Verfahren nach Anspruch 6, **dadurch gekennzeichnet, daß** das ascorbinsäurehaltige flüssige Lösungsmittel im Gleichstrom mit Luft sprühgetrocknet wird.

## Claims

1. Vitamin C preparation comprising microparticles of substantially pure ascorbic acid, **characterised in that** the microparticles are in the form of small bubbles or hollow spherules, the diameter of which is smaller than 0.2mm, and having a ratio of solid mass to surface area less than 50% of this ratio with crystalline ascorbic acid.

2. Vitamin C preparation as claimed in claim 1, **characterised in that** the ascorbic acid bubbles are filled with gas.

3. Vitamin C preparation as claimed in claim 1, **characterised in that** the bubbles or hollow spherules have a diameter less than 0.1mm.

4. Vitamin C preparation as claimed in claim 1, **characterised in that** a plurality of bubbles or hollow spherules are granulated to form larger aggregates whilst substantially maintaining their bubble or hollow spherule shape.

5. Vitamin C preparation as claimed in claim 4, **characterised in that** the granulates have a maximum diameter of less than 0.5mm.

6. Method of manufacturing a vitamin C preparation as claimed in one of claims 1 to 5, **characterised in that** crystalline ascorbic acid is dissolved in a liquid solvent and then subjected to spray drying, whereby the solution is sprayed very finely and the liquid solvent is evaporated, that fine bubbles or hollow spherules formed thereafter upon are collected and introduced into containers in a substantially air-tight and light shielded manner.

7. Method as claimed in claim 6, **characterised in that** the fine bubbles or hollow spherules produced in the spray drying are combined into larger aggregates before the packaging, whereby they are fused together at their outer skins.

8. Method as claimed in claim 7, **characterised in that** the fine ascorbic acid bubbles or hollow spherules are agglomerated by the addition of a bonding agent.

9. Method as claimed in claim 8, **characterised in that** the ascorbic acid bubbles or hollow spherules are sprayed with a starch solution for the agglomeration.

10. Method as claimed in one of claim 6 to 9, **characterised in that** the crystalline ascorbic acid is dissolved in an aqueous solvent.

11. Method as claimed in claim 10, **characterised in that** distilled water is used as the aqueous solvent.

12. Method as claimed in claim 6, **characterised in that** the ascorbic acid-containing liquid solvent is spray dried in co-current with air.

## Revendications

1. Préparation de vitamine C constituée de microparticules d'acide ascorbique pratiquement pur, **caractérisée par le fait que** les microparticules sont des petites bulles ou des petites boules creuses de diamètre inférieur à 0,2 mm et ont un rapport de la masse solide à la surface solide inférieur à 50 % de ce rapport pour l'acide ascorbique cristallin.

2. Préparation de vitamine C selon la revendication 1, **caractérisée par le fait que** les petites bulles ou petites boules creuses d'acide ascorbique sont remplies de gaz.

3. Préparation de vitamine C selon l'une des revendications 1 et 2, **caractérisée par le fait que** les petites bulles ou petites boules creuses ont un diamètre inférieur à 0,1 mm.

4. Préparation de vitamine C selon l'une des revendications 1 à 3, **caractérisée par le fait que** plusieurs petites bulles ou petites boules creuses sont granulées en agrégats plus gros en conservant dans une large mesure leur forme.

5. Préparation de vitamine C selon la revendication 4, **caractérisée par le fait que** les granulés ont un diamètre maximal inférieur à 0,5 mm.

6. Procédé d'élaboration d'une préparation de vitamine C selon l'une des revendications 1 à 5, **caractérisé par le fait qu'**on dissout de l'acide ascorbique cristallin dans un solvant liquide et ensuite le soumet à un séchage par pulvérisation en pulvérisant la solution de manière très fine et évapore le solvant liquide, et qu'ensuite, on recueille les petites bulles ou petites boules creuses fines obtenues et les enferme dans des récipients de manière pratiquement étanche à l'air et à l'abri de la lumière.

7. Procédé selon la revendication 6, **caractérisé par le fait qu'**avant de mettre en récipients les petites bulles ou petites boules creuses fines produites lors du séchage par pulvérisation, on les réunit en agrégats plus gros en les faisant fusionner à leurs peaux extérieures.

8. Procédé selon la revendication 7, **caractérisé par le fait qu'**on agglomère les petites bulles ou petites boules creuses fines d'acide ascorbique en y ajoutant un liant.

9. Procédé selon la revendication 8, **caractérisé par le fait que** pour agglomérer les petites bulles ou petites boules creuses d'acide ascorbique, on les arrose d'une solution d'amidon.

10. Procédé selon les revendications 6 à 9, **caractérisé par le fait qu'**on dissout l'acide ascorbique cristallin dans un solvant aqueux.

11. Procédé selon la revendication 10, **caractérisé par le fait qu'**on utilise comme solvant aqueux de l'eau distillée.

12. Procédé selon la revendication 6, **caractérisé par le fait qu'**on élimine le solvant liquide contenant de l'acide ascorbique par séchage par pulvérisation à l'air en courants parallèles.
